# EUROPEAN PATENT APPLICATION

(11) **EP 2 305 129 A2**
(43) Date of publication of application: **06.04.2011**
(21) Application number: 10251691.1
(22) Date of filing: 30.09.2010
(51) Int. Cl.: A61B 17/04, A61B 17/00

(54) **Wound closure device including ferrule ejector systeme**

(30) Priority: 01.10.2009 US 247650 P; 22.09.2010 US 887766
(71) Applicant: Tyco Healthcare Group LP, New Haven, CT 06511 (US)
(72) Inventor: Smith, Robert C., Middletown, CT 06457 (US)
(74) Representative: Soames, Candida Jane

(57) **Abstract**

A suturing device (10) includes a housing having a shaft (200) attached thereto and one or more guide lumens (110,120) extending therethrough. The shaft has a head portion disposed at a distal end thereof. The head portion is configured to retain a portion of a suture therein. A ferrule assembly (400) is rotatably coupled to the shaft and includes at least one ferrule holder. Each ferrule holder (430,440) is configured to releasably retain a ferrule (435,445) therein. Each ferrule is configured to retain a portion of the suture (600) thereon. The ferrule assembly is rotatable between a first position and a second position. The guide lumen(s) are configured to direct a suture passer (500) into engagement with the ferrule of one of the ferrule holders when the ferrule assembly is in one of the first and second positions.

## Description

### CROSS-REFERENCE TO RELATED APPLICATION

The present application claims the benefit of and priority to U.S. Provisional Application Serial. No. 61/247,650 filed on October 1, 2009, the entire contents of which are incorporated by reference herein.

### BACKGROUND

### Technical Field

The present disclosure relates to a wound closure device and, more particularly, to a wound closure device having a ferrule ejector system for suturing a wound.

### Description of Related Art

Puncture wounds, wounds that pierce through tissue, may result from trauma or may be intentionally created in order to provide access to a body cavity during surgical procedures. During endoscopic surgical procedures, for example, a trocar device is utilized to puncture the peritoneum to provide an access port by way of a cannula through the abdominal wall. Generally, a trocar and/or cannula is placed through the abdominal wall for introduction of surgical instrumentation which is necessary to carry out the surgical procedure. In this manner, the surgeon may introduce a surgical instrument such as a grasper, scissor, clip applier, stapler or any other surgical instrument which may be necessary during the particular surgical procedure. Once the procedure is complete, it is necessary to suture the wound.

Conventional instruments for closing puncture wounds generally include a shaft that can be extended into the body through either the puncture wound itself (in the case of a puncture caused by trauma) or through a cannula (in the case of a puncture created to access a surgical site). Suture retaining needles are then deployed from the shaft into tissue. Unfortunately, the mechanisms used for deploying the needles are often cumbersome and may make the extension and/or retraction of the suturing device difficult.

In the prior art, U.S. Patent No. 5,470,330 discloses a suturing instrument for closing trocar puncture wounds. The suturing instrument includes a pair of needle carriers having needles releasably retained thereon. The needle carriers are translatable from a retracted position to a deployed position to urge the needles into tissue. U.S. Patent No. 6,296,640 discloses a suturing aid including one or more vanes that are moveable from a first position, wherein the vanes are folded up proximally for insertion, to a second position, wherein the vanes are swung out laterally to help align a suturing instrument, and, finally, to a third position, wherein the vanes are folded up distally for removal. U.S. Patent No. 6,911,034 discloses a suturing apparatus including one or more arms having a suture mounting portion and one or more needles. Each arm is extendable from the apparatus to penetrate tissue, while each needle is advanceable to a position adjacent an arm to capture the suture mounted thereon and to draw the suture back toward the suturing apparatus. U.S. Patent No. 7,235,087 discloses an articulating suturing device having an articulating foot. The foot includes suture attachment cuffs configured to engage needles for withdrawing the cuffs through tissue. U.S. Patent No. 7,449,024 discloses a suturing device having arms that are extendable from the suturing device. Needles may then be advanced into engagement with the arms to retrieve a suture from the arms. U.S. Patent Application Publication No. 2006/0030868 discloses a suturing device including a pair of wings that are selectively extendable from the device. Needles carrying sutures thereon may then be advanced through tissue and into engagement with the wings to retain the sutures thereon. U.S. Patent Application Publication Nos. 2008/0045979 and 2009/0157105 disclose articulating suture devices similar to that disclosed in U.S. Patent No. 7,235,087, discussed above.

### SUMMARY

In accordance with the present disclosure, a suturing device is provided. The suturing device includes a housing having a shaft attached thereto and one or more guide lumens extending therethrough. The shaft includes a head portion disposed at a distal end thereof. The head portion is configured to retain a portion of a suture therein. A ferrule assembly is rotatably coupled to the shaft and includes one or more ferrule holders each of which is configured to releasably retain a ferrule therein. Each ferrule is configured to retain a portion of the suture thereon. The ferrule assembly is rotatable with respect to the shaft between a first position and a second position. Each guide lumen is configured to direct a suture passer inserted therethrough into engagement with the ferrule of one of the ferrule holders when the ferrule assembly is disposed in the first position.

In one embodiment, the suturing device further includes a manually manipulatable member operably coupled to the ferrule assembly. The manually manipulatable member is configured to rotate the ferrule assembly between the first and second positions.

The ferrule assembly may be positioned substantially perpendicular to the shaft when in the first position such that the ferrule holders are radially spaced from the shaft when in the first position. Further, the ferrule assembly may be positioned substantially parallel to the shaft when in the second position such that the ferrule holders are disposed within an outer diameter of the shaft when in the second position.

In another embodiment, the suture passer includes a needle configured to engage the ferrule in a female-male friction fit engagement.

In yet another embodiment, the ferrule assembly includes a rotatable plate having first and second ferrule holders disposed at first and second ends, respectively, thereof. The first and second ferrule holders may be configured for positioning on opposite sides of a wound in tissue when moved to the first position.

In still yet another embodiment, the ferrule assembly is biased toward the first position.

### BRIEF DESCRIPTION OF THE DRAWINGS

Various embodiments of the subject instrument are described herein with reference to the drawings wherein:

Fig. 1 is a perspective view of a suturing device according to the present disclosure with a portion of the housing removed and with a ferrule assembly in a first position;

Fig. 2 is a top, cross-sectional view of the suturing device of Fig. 1;

Fig. 3 is an enlarged view of one end of the ferrule assembly of the suturing device of Fig. 1; and

Fig. 4 is a top view of the suturing device of Fig. 1, with the ferrule assembly in a second position.

### DETAILED DESCRIPTION

In the figures and in the description that follows, in which like reference numerals identify similar or identical elements, the term "proximal" will refer to the end of the apparatus which is closest to the operator during use, while the term "distal" will refer to the end which is farthest from the operator, as is traditional.

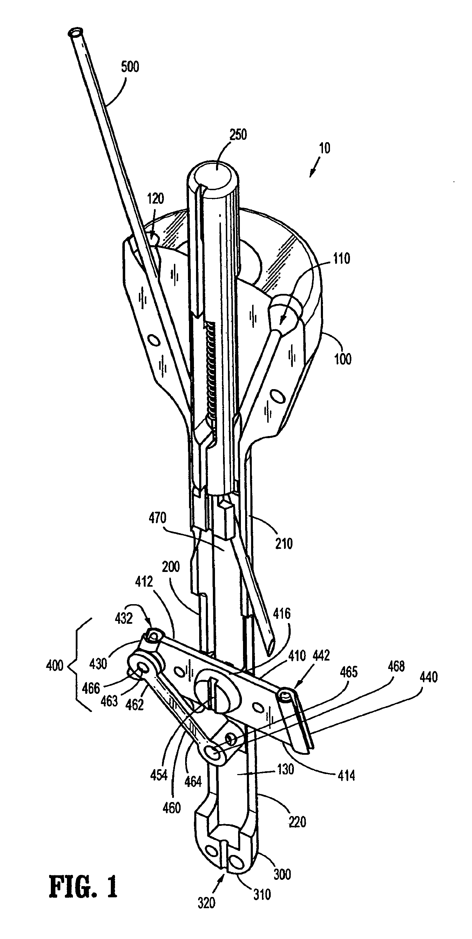

Referring now to Figs. 1-4, suturing device 10 is shown generally including a housing 100, a shaft 200, a head portion 300 and a ferrule assembly 400. Shaft 200 is attached at a proximal end 210 to housing 100 and at a distal end 220 to head portion 300. Ferrule assembly 400 is disposed on the shaft 200 and is configured to rotate between a first position and a second position with respect to shaft 200, as will be described in greater detail below. A manually manipulatable member, or plunger 250, is coupled to ferrule assembly 400 and is selectively translatable to rotate ferrule assembly 400 between the first and second positions. A suture passer, or needle 500, is configured for insertion through housing 100 via guide lumens 110, 120. Each guide lumen 110, 120 directs needle 500 toward a corresponding ferrule holder 430, 440 of rotating plate 410 of ferrule assembly 400 when ferrule assembly 400 is disposed in the second position. Needle 500 includes a distal tip portion 510 and a lumen 520 extending at least partially therethrough.

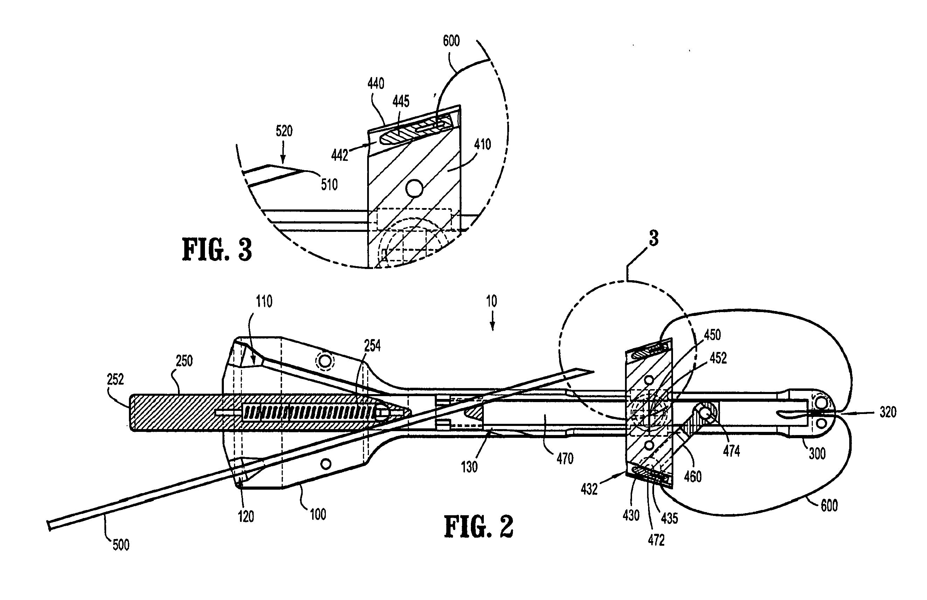

As mentioned above, head portion 300 is disposed at a distal end 220 of shaft 200. Distal end 310 of head portion 300 may be generally conically shaped to facilitate the insertion of suturing device 10 through an opening in tissue and/or may include a blunt tip portion to help avoid damaging tissue upon insertion into an opening in tissue. Lumen 320 of head portion 300 is configured to releasably retain a portion of a suture 600 therein. As shown in Figs. 2 and 3, both ends of suture 600 extend from lumen 320. More particularly, one end of suture 600 is attached to ferrule 435 (Fig. 2) while the other end of suture 600 is attached to ferrule 445 (Fig 3). Each ferrule 435, 445 is removably disposed in a respective ferrule lumen 432, 442 of a ferrule holder 430, 440 of rotating plate 410.

Rotating plate 410 includes two ferrule holders 430, 440 disposed at opposite ends 412, 414 of rotating plate 410, respectively. Each ferrule holder 430, 440 includes a respective ferrule lumen 432, 442 for releasably retaining a ferrule 435, 445, respectively, therein. Ferrule lumens 432 and 442 extend through rotating plate 410, as shown in Figs. 1-3. It is envisioned that rotating plate 410 may include more than two ferrule lumens extending therethrough and it is also contemplated that the ferrule lumens 432, 442 may be disposed at different positions along rotating plate 410. Alternatively, the ferrule holders 430, 440 may include any other suitable structure capable of releasably retaining a ferrule 435, 445 thereon.

Rotating plate 410 further includes a central aperture 450 defined through a central portion 416 thereof. A pivot pin 452 is disposed through central aperture 450 of rotating plate 410 and is retained therethrough via nut 454 disposed on an opposide side of rotating plate 410. Pivot pin 452 is similarly disposed through an aperture (not explicitly shown) defined within shaft 200 thereby securing rotating plate 410 on shaft 200 such that rotating plate 410 may rotate about pivot pin 452 with respect to shaft 200, e.g., between first and second positions. Arm 460 includes first and second ends 462 and 464, respectively, which have apertures 463 and 465, respectively, defined therethrough. Pivot pin 466 connects first end 462 of arm 460 with rotating plate 410. More particularly, pivot pin 466 is disposed through both aperture 463 of first end 462 of arm 460 and aperture 472 of end 412 of rotating plate 410 such that rotating plate 410 is rotatable with respect to arm 460 about pivot 466. Similarly, pivot pin 468 connects second end 464 of arm 460 with bar 470 via apertures 465 and 474, respectively. Bar 470 is longitudinally translatable through conduit 130 of shaft 200, as will be discussed in greater detail below.

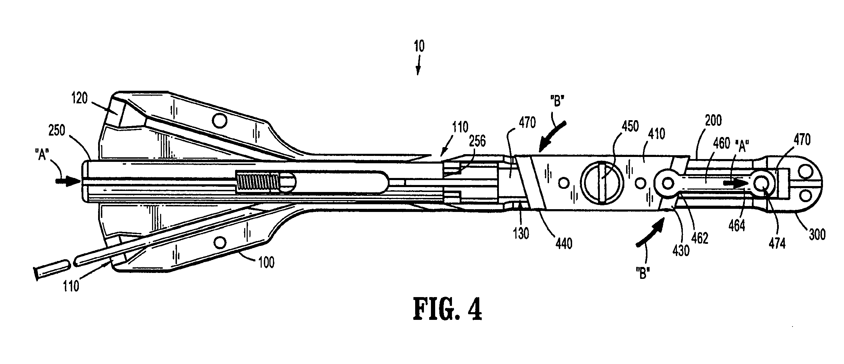

As shown in Figs. 1-4, plunger 250 is disposed at least partially through conduit 130 of housing 100. Conduit 130 extends distally from housing 100 through shaft 200. Proximal end 252 of plunger 250 extends proximally from housing 110 and is selectively depressible by an operator to translate plunger 250 distally through conduit 130 to the position shown in Fig. 4. Upon distal translation of plunger 250, i.e., when an operator depresses plunger 250, distal end 256 of plunger 250 repositions, or translates bar 470 distally. Spring 254 is configured to bias plunger 250 toward the position shown in Figs. 1 and 2, e.g., a proximal-most position. Thus, in order to depress plunger 250 and translate bar 470 distally, the operator must overcome the biasing force of spring 254.

Initially, when plunger 250 is disposed in an at-rest, or proximal-most position, rotating plate 410 is disposed in the first position, wherein base plate 410 is positioned substantially perpendicular to shaft 200 such that ferrule holders 430, 440 extend radially outward from shaft 200, as shown in Figs. 1-3. This first position corresponds to the use position of suturing device 10, as will be described in greater detail below.

When plunger 250 is depressed distally, e.g., in the direction of arrow "A," as shown in Fig. 4, bar 470 is urged distally through conduit 130 by distal tip 256 of plunger 250. Accordingly, arm 460, which is rotatably secured to bar 470 at second end 464 via pivot pin 468, is pulled distally in the direction of arrow "A" with respect to shaft 200. As arm 460 is pulled distally, rotating plate 410 is rotated in the direction of arrow "B" due to the pivotable connection of end 412 of rotating plate 410 with end 462 of arm 460. Upon further distal translation of plunger 250, and thus bar 470, rotating plate 410 is rotated to the position shown in Fig. 4. In this second position, rotating plate 410 is aligned with shaft 200 such that rotating plate 410 does not extend beyond the dimensions of shaft 200, i.e., such that rotating plate 410 is positioned substantially parallel with respect to shaft 200. As such, in this second position, or insertion position, suturing device 10 defines a minimum diameter to facilitate insertion through an opening in tissue. It is also contemplated that suturing device 10 include a locking mechanism (not shown) configured to lock rotating plate 410 in one or both of the first and second positions.

In preparation for suturing, a middle portion of suture 600 is retained within lumen 320 of head portion 300. One end of suture 600 is then attached to ferrule 435, while the other end of suture 600 is attached to ferrule 445. Next, ferrules 435 and 445 and loaded into the ferrule lumens 432 and 442 of ferrule holders 430 and 440, respectively, of ferrule assembly 400. At this point, suturing device 10 is configured, or loaded for use. Once suturing device 10 is loaded for use, plunger 250 may be depressed to rotate rotating plate 410 to the second, or insertion position, as shown in Fig. 4 such that suturing device 10 may then be inserted into an opening in tissue.

Suturing device 10 is inserted, starting with head portion 300, through an opening (wound) in tissue that is to be sutured. The suturing device 10 is translated through the wound until the ferrule assembly 400 is disposed adjacent an interior surface of tissue to be sutured. Once positioned accordingly, plunger 250 may be released, thereby allowing plunger 250 and, thus bar 470, to translate proximally back to the first, or at-rest position, e.g., the use position, as shown in Figs. 1 and 2. More specifically, as bar 470 is translated proximally, rotatable plate 410 is rotated to the first position such that ferrule lumen 432 of ferrule holder 430, which houses ferrule 435, is disposed adjacent a first side of the wound and such that ferrule lumen 442 of ferrule holder 440, which houses ferrule 445, is disposed adjacent an opposite side of the wound. Thus, it is envisioned that a length of rotating plate 410 be configured according to the diameter of wound to be sutured. It is also envisioned that rotating plate 410 and arm 460 are removable from suturing device 10 such that an operator may select a rotatable plate and corresponding arm having a length sufficient to allow the ferrule holders 430, 440 to be disposed on either side of the wound when in the first position.

Continuing with the operation of suturing device 10, once ferrule assembly 400 is inserted through the wound in tissue and is moved back to the first position, wherein ferrule holders 430 and 440 and, thus, ferrule lumens 432 and 442 are disposed adjacent opposing sides of the wound, needle 500 is inserted through guide lumen 120 of housing 100, as shown in Figs. 1 and 2. As needle 500 is translated distally through guide lumen 120, guide lumen 120 directs needle 500 through tissue and toward ferrule 445 due to the alignment of guide lumen 120 and ferrule holder 440, when ferrule assembly 400 is in the first position. Upon further distal translation of needle 500, distal tip 510 of needle 500 passes completely through tissue and eventually enters ferrule lumen 442 of ferrule holder 440. As needle 500 is urged distally into lumen 442, needle 500 surrounds ferrule 445 such that ferrule 445 is disposed through lumen 520 of needle 500. Lumen 520 may have a slightly smaller diameter than ferrule 445 such that when needle 500 is urged around ferrule 445, ferrule 445 becomes lodged within lumen 520, fixedly retaining ferrule 445 therein via a male-female friction-fit engagement. Alternatively, lumen 520 may taper proximally from distal tip 510 from a first diameter which is larger than the diameter of ferrule 445 to a second diameter which is smaller than the diameter of ferrule 445. In this configuration, further urging of needle 500 around ferrule 445 engages ferrule 445 within lumen 520 in a male-female friction-fit engagement. In another alternative embodiment, needle 500 may define a sufficiently small diameter to engage a lumen (not shown) defined in ferrule 445. In this embodiment, needle 500 may be urged into the lumen defined in ferrule 445 such that the ferrule 445 and needle 500 are engaged in a male-female friction-fit engagement. While described with reference to ferrule 445, it is envisioned that any of the above-mentioned configurations of needle 500 and ferrule 445 may be similarly used with respect to ferrule 435 and/or a different suture passer 500. It is also envisioned that other suitable mechanisms or configurations for engaging needle 500 and ferrules 435, 445 may be provided.

Once ferrule 445 is retained within lumen 520 of needle 500, needle 500 may be pulled proximally, or retracted from ferrule holder 440. As needle 500 is pulled further proximally back through tissue, ferrule 445 is removed from ferrule lumen 442. Needle 500 and, thus, ferrule 445 and suture 600, are translated proximally through tissue until needle 500 and ferrule 445 have passed completely through tissue, leaving suture 600 disposed through tissue on one side of the wound. More specifically, the end of suture 600 engaged to ferrule 445 extends proximally through tissue, while the middle portion of suture 600 remains disposed within head assembly 300, which is disposed within tissue, i.e., on a distal side of tissue. Needle 500 is then removed from guide lumen 120 and ferrule 445 is separated from needle 500.

Next, needle 500, or a new needle substantially similar to needle 500, is inserted into guide lumen 110 of housing 100. Guide lumen 110 directs needle 500 through tissue on the opposing side of the wound and toward ferrule holder 430. Similarly as described above, needle 500 is then translated further through ferrule lumen 432 of ferrule holder 440 to engage ferrule 435 therein in a male-female friction-fit engagement. Needle 500 and ferrule 435 are then translated proximally back through tissue, as described above, such that suture 600 is disposed through tissue on an opposing side of the wound. Needle 500 may then be removed from the wound and plunger 250 may be depressed to rotate rotating plate 410 to the second position, as described above. Suturing instrument 10 may then be removed from the wound, leaving suture 600 disposed through tissue on both sides of the wound with the middle portion of suture 600 interconnecting the ends thereof on a distal, or internal side of tissue. Head assembly 300 may be configured to release suture 600 therefrom upon removal of suturing instrument 10 from the wound in tissue, or suture 600 may be manually released from head assembly 300 upon removal of suturing instrument 10. Once suturing instrument 10 has been removed, the operator can then tighten and tie off the free ends of suture 600 to close the wound.

From the foregoing and with reference to the various figure drawings, those skilled in the art will appreciate that certain modifications can also .be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A suturing device, comprising:
a housing having a shaft attached thereto and defining at least one guide lumen extending therethrough, the shaft having a head portion disposed at a distal end thereof, the head portion configured to retain a portion of a suture therein; and
a ferrule assembly rotatably coupled to the shaft, the ferrule assembly including at least one ferrule holder disposed thereon, each ferrule holder being configured to releasably retain a ferrule therein, each ferrule being configured to retain a portion of the suture thereon, the ferrule assembly being rotatable with respect to the shaft between a first position and a second position, wherein the at least one guide lumen is configured to direct a suture passer inserted therethrough into engaged with the ferrule of one of the ferrule holders when the ferrule assembly is disposed in one of the first and second positions.

2. The suturing device of claim 1, wherein the housing further includes a manually manipulatable member operably coupled to the ferrule assembly, the manually manipulatable member configured to rotate the ferrule assembly between the first and second positions.

3. The suturing device according to claim 1 or claim 2, wherein the ferrule assembly is positioned substantially perpendicular to the shaft when in the first position such that the ferrule holders are radially spaced from the shaft when in the first position.

4. The suturing device according to claim 1 or claim 2, wherein the ferrule assembly is positioned substantially parallel to the shaft when in the second position such that the ferrule holders are disposed within an outer diameter of the shaft when in the second position.

5. The suturing *device* according to any preceding claim, wherein the suture passer comprises a needle configured to engage the ferrule in a female-male friction fit engagement.

6. The suturing device according to any preceding claim, wherein the ferrule assembly includes a rotatable plate having first and second ferrule holders disposed at first and second ends thereof.

7. The suturing *device* according to claim 6, wherein the first and second ferrule holders are positionable on opposite sides of a wound when *moved* to the first position.

8. The suturing device according to any preceding claim, wherein the ferrule assembly is biased toward the first position.
